# EUROPEAN PATENT SPECIFICATION

(11) **EP 3 919 509 B1**
(45) Date of publication and mention of the grant of the patent: **08.10.2025**
(21) Application number: 20747633.4
(22) Date of filing: 30.01.2020
(51) Int. Cl.: C07K 16/18, C12N 15/13, G01N 33/53

(54) **METHOD FOR IMMUNOLOGICAL ANALYSIS OF FREE AIM IN BIOLOGICAL SAMPLE**
VERFAHREN ZUR IMMUNOLOGISCHEN ANALYSE VON FREIEM AIM IN EINER BIOLOGISCHEN PROBE
PROCÉDÉ D'ANALYSE IMMUNOLOGIQUE D'AIM LIBRE DANS UN ÉCHANTILLON BIOLOGIQUE

(30) Priority: 31.01.2019 JP 2019015199
(43) Date of publication of application: 08.12.2021
(73) Proprietor: Sekisui Medical Co., Ltd., Tokyo 103-0027 (JP); Miyazaki, Toru, Tokyo 1120003 (JP); Social Welfare Organization "Saiseikai" Imperial Gift Foundation Inc., Tokyo 108-0073 (JP)
(72) Inventor: MIYAZAKI, Toru, Tokyo 112-0003 (JP); YAMAZAKI, Tomoko, 3-1 Hongo 7-chome Bunkyo- ku, Tokyo 113-0033 (JP); OKANOUE, Takeshi, Suita-shi, Osaka 564-0013 (JP); ASAI, Tomohide, Tokyo 103-0027 (JP); KANETSUKI, Yuka, Tokyo 103-0027 (JP); HIROTA, Jiro, Tokyo 103-0027 (JP)
(74) Representative: Hoffmann Eitle
(86) International application number: PCT/JP2020/003414
(87) International publication number: WO 2020/158856

(56) References cited:
- EP-A1- 3 919 906
- WO-A1-2017/022315
- WO-A1-2017/043617
- "Human AIM/ CD 5L/Spa ELISA Kit User's Manual", MBL, Retrieved from the Internet <URL:https://www.mblintl.com/assets/CY-8080-v141003.pdf> [retrieved on 20200313]
- ARAI, S. ET AL.: "Apoptosis inhibitor of macrophage protein enhances intraluminal debris clearance and ameliorates acute kidney injury in mice", NATURE MEDICINE, vol. 22, no. 2, 4 January 2016 (2016-01-04), pages 183 - 193, XP055362476, DOI: 10.1038/nm.4012
- MIYAZAKI, T. ET AL.: "AIM associated with the IgM pentamer: attackers on stand-by at aircraft carrier", CELLULAR & MOLECULAR IMMUNOLOGY, vol. 15, 29 January 2018 (2018-01-29), pages 563 - 574, XP036733609, DOI: 10.1038/cmi.2017.141
- ARAI, S. ET AL.: "Obesity-Associated Autoantibody Production Requires AIM to Retain the Immunoglobulin M Immune Complex on Follicular Dendritic Cells", CELL REPORTS, vol. 3, no. 4, 2013, pages 1187 - 1198, XP009172535, DOI: 10.1016/j.celrep.2013.03.006
- HIRAMOTO, E. ET AL.: "The IgM pentamer is an asymmetric pentagon with an open groove that binds the AIM protein", SCIENCE ADVANCES, vol. 4, no. 10, 10 October 2018 (2018-10-10), XP055729532, Retrieved from the Internet <URL:https://advances.sciencemag.org/content/4/10/eaaull99> [retrieved on 20200313]

## Description

### TECHNICAL FIELD

The present invention relates to an immunoassay method for free AIM in a biological sample and to an anti-AIM monoclonal antibody having a property of reacting with free AIM and not reacting with complex AIM. The present invention also relates to an assay kit for free AIM in a biological sample comprising the monoclonal antibody.

### BACKGROUND ART

AIM (apoptosis inhibitor of macrophage) is a secretory blood protein with a molecular weight of about 50 kDa produced by tissue macrophages. AIM has a structure in which three scavenger recipient cysteine-rich (SRCR) domains (SRCR1 domain, SRCR2 domain, and SRCR3 domain) having specific sequences containing many cysteine residues are connected in tandem, and the cysteine residues are considered to be disulfide-bonded to each other in each domain to form a compact spherical three-dimensional structure.

AIM is known to have the characteristic of binding to various molecules such as lipopolysaccharide, IgM, complement regulatory factors, and fatty acid synthetases. Particularly, AIM is known to exist in the form of a complex with IgM in the blood. Since IgM is a huge protein complex exceeding 500 kDa, AIM does not pass through the glomerulus and transfer to urine as long as AIM is bound to IgM, and a high blood concentration of AIM is maintained. When dissociated from IgM, AIM is promptly excreted into urine. Therefore, most of AIM forms a complex with IgM in the blood and are rarely present in the blood in a free state rather than as a conjugate.

In recent years, it has been clarified that AIM is involved in the progression of pathological conditions in various diseases such as insulin resistance or arteriosclerosis. For example, Patent Document 1 reports a relationship between free AIM present in a free form not bound to another binding partner and liver disease.

### CITATION LIST

### PATENT LITERATURE

Patent Document 1: WO 2017/043617 relates to a method for examining liver cancer or a method for assisting diagnosis of liver cancer as well as a kit that can be used for this purpose.

### SUMMARY OF INVENTION

### TECHNICAL PROBLEM

When a specific disease is diagnosed based on a measurement result of a free AIM amount, it is necessary to eliminate an amount of AIM forming a complex and measure only the free AIM amount. However, when free AIM is detected, complex AIM bound to another binding partner may also be detected. Therefore, a demand exists for a technique capable of eliminating an amount of AIM forming a complex and measuring only an amount of free AIM without complicated operations.

An object of the present invention is to provide an immunoassay method for free AIM in a biological sample containing complex AIM and free AIM and an assay kit with excellent specificity and sensitivity, and an anti-AIM monoclonal antibody specifically reacting with free AIM with excellent specificity and sensitivity.

### SOLUTION TO PROBLEM

As a result of intensive studies for solving the problem, the present inventor produced an anti-AIM monoclonal antibody reacting with free AIM without reacting with complex AIM, and an anti-AIM monoclonal antibody reacting with both complex AIM and free AIM. The present inventor then found that the anti-AIM monoclonal antibody reacting with free AIM without reacting with complex AIM recognizes the SRCR2 domain of AIM as an epitope, thereby completing the present invention.

The present invention provides the immunoassay methods, the monoclonal antibodies, and the kits as defined by the appended claims.

### ADVANTAGEOUS EFFECTS OF INVENTION

According to the present invention, free AIM can accurately be detected in a biological sample containing complex AIM and free AIM.

### BRIEF DESCRIPTION OF DRAWINGS

[Fig. 1A] Fig. 1A is a diagram showing a result of study on combination of monoclonal antibodies in an AIM measurement ECL system.
[Fig. 1B] Fig. 1B is a diagram showing a result of a study on the combination of monoclonal antibodies in the ECL method for AIM measurement.
[Fig. 1C] Fig. 1C is a diagram showing a result of a study on the combination of monoclonal antibodies in the ECL method for AIM measurement.
[Fig. 1D] Fig. 1D is a diagram showing a result of a study on the combination of monoclonal antibodies in the ECL method for AIM measurement.
[Fig. 1E] Fig. 1E is a diagram showing a result of a study on the combination of monoclonal antibodies in the ECL method for AIM measurement.
[Fig. 1F] Fig. 1F is a diagram showing a result of a study on the combination of monoclonal antibodies in the ECL method for AIM measurement.
[Fig. 1G] Fig. 1G is a diagram showing a result of a study on the combination of monoclonal antibodies in the ECL method for AIM measurement.
[Fig. 1H] Fig. 1H is a diagram showing a result of a study on the combination of monoclonal antibodies in the ECL method for AIM measurement.
[Fig. 2] Fig. 2 is a diagram showing a result of epitope identification analysis of monoclonal antibodies by western blotting.

### DESCRIPTION OF EMBODIMENTS

### (Biological Sample)

Examples of a biological sample analyzable in the present invention include solid tissues and body fluids derived from living bodies (organisms), and body fluids are preferably used. The biological sample analyzable in the present invention is more preferably a body fluid sample such as blood, serum, plasma, urine, saliva, sputum, tear fluid, otorrhea, or prostatic fluid, further preferably blood, serum, plasma, or urine. Examples of the living body or the subject include humans or animals (e.g., mice, guinea pigs, rats, monkeys, dogs, cats, hamsters, horses, bovines, and pigs), and are preferably humans. The biological sample from the subject may be collected or prepared at the time of implementation of the present invention or may preliminarily be collected or prepared and stored. The person preparing the sample and the person analyzing the amount of free AIM in the sample may be different. The biological sample can be an in vivo sample. In the present invention, the biological sample contains both free AIM and complex AIM.

### (AIM)

AIM (apoptosis inhibitor of macrophage) is a secretory blood protein with a molecular weight of about 50 kDa produced by tissue macrophages. AIM has a structure in which three scavenger recipient cysteine-rich (SRCR) domains, i.e., specific sequences containing many cysteine residues, are connected in tandem, and the cysteine residues are considered to be disulfide-bonded to each other in each domain to form a compact spherical three-dimensional structure.

Human AIM is composed of 347 amino acids represented by SEQ ID NO: 1 and contains three SRCR domains rich in cysteine. The SRCR1 domain corresponds to amino acid numbers 24 to 125 in the amino acid sequence represented by SEQ ID NO: 1. The SRCR2 domain corresponds to amino acid numbers 138 to 239 in the amino acid sequence represented by SEQ ID NO: 1. The SRCR3 domain corresponds to amino acid numbers 244 to 346 in the amino acid sequence represented by SEQ ID NO: 1.

The amino acid sequence of human AIM is as follows.

MALLFSLILAICTRPGFLASPSGVRLVGGLHRCEGRVEVEQKGQWGTVCDDGWD
IKDVAVLCRELGCGAASGTPSGILYEPPAEKEQKVLIQSVSCTGTEDTLAQCEQEE
VYDCSHDEDAGASCENPESSFSPVPEGVRLADGPGHCKGRVEVKHQNQWYTVC
QTGWSLRAAKVVCRQLGCGRAVLTQKRCNKHAYGRKPIWLSQMSCSGREATLQ
DCPSGPWGKNTCNHDEDTWVECEDPFDLRLVGGDNLCSGRLEVLHKGVWGSV
CDDNWGEKEDQVVCKQLGCGKSLSPSFRDRKCYGPGVGRIWLDNVRCSGEEQS
LEQCQHRFWGFHDCTHQEDVAVICSG (SEQ ID NO: 1)

Specifically, the amino acid sequences of the SRCR1 domain, the SRCR2 domain, and the SRCR3 domain in human AIM are as follows.
SRCR1 domain: 
VRLVGGLHRCEGRVEVEQKGQWGTVCDDGWDIKDVAVLCRELGCGAASGTPSG
ILYEPPAEKEQKVLIQSVSCTGTEDTLAQCEQEEVYDCSHDEDAGASCE (SEQ ID
NO: 2)
SRCR2 domain: 
VRLADGPGHCKGRVEVKHQNQWYTVCQTGWSLRAAKVVCRQLGCGRAVLTQ
KRCNKHAYGRKPIWLSQMSCSGREATLQDCPSGPWGKNTCNHDEDTWVECE
(SEQ ID NO: 3)
SRCR3 domain:

LRLVGGDNLCSGRLEVLHKGVWGSVCDDNWGEKEDQVVCKQLGCGKSLSPSF
RDRKCYGPGVGRIWLDNVRCSGEEQSLEQCQHRFWGFHDCTHQEDVAVICS
(SEQ ID NO: 4)

### (Free AIM)

In this description, the "free AIM" means AIM existing in a free state without being bound to other substances such as lipopolysaccharide or IgM. On the other hand, in this description, AIM bound to other substances such as lipopolysaccharide or IgM and existing in a state of a complex is referred to as complex AIM. The free AIM is preferably human free AIM, and the complex AIM is preferably human complex AIM.

In this description, "non-specific reaction" means that a substance other than free AIM, particularly complex AIM, binds to an anti-AIM monoclonal antibody used in the present invention.

### (Anti-AIM Monoclonal Antibody Having Property of Reacting with Free AIM and Not Reacting with Complex AIM)

An immunoassay method of the present invention uses an anti-AIM monoclonal antibody having a property of reacting with free AIM and not reacting with complex AIM. "Reacting with free AIM and not reacting with complex AIM" means reacting with free AIM and substantially not reacting with other substances, particularly complex AIM. The meaning of "substantially not reacting" will be described later. Hereinafter, the "anti-AIM monoclonal antibody having a property of reacting with free AIM and not reacting with complex AIM" may be referred to as an "anti-AIM monoclonal antibody specifically reacting with free AIM". In this description, in contrast with the anti-AIM monoclonal antibody specifically reacting with free AIM, an anti-AIM monoclonal antibody reacting with both free AIM and complex AIM is referred simply as an "anti-AIM monoclonal antibody".

In the present invention, "using an anti-AIM monoclonal antibody having a property of reacting with free AIM and not reacting with complex AIM" means quantifying free AIM by using the anti-AIM monoclonal antibody having a property of reacting with free AIM and not reacting with complex AIM. "Using an anti-AIM monoclonal antibody having a property of reacting with free AIM and not reacting with complex AIM" can include the following steps.

A step of bringing the anti-AIM monoclonal antibody having a property of reacting with free AIM and not reacting with complex AIM into contact with a biological sample.

In the immunoassay method of the present invention, two or more anti-AIM monoclonal antibodies having a property of reacting with free AIM and not reacting with complex AIM are preferably used. The two or more anti-AIM monoclonal antibodies preferably recognize respective different epitopes.

In the immunoassay method of the present invention, in addition to the anti-AIM monoclonal antibody having a property of reacting with free AIM and not reacting with complex AIM, an anti-AIM monoclonal antibody having a property of reacting with both free AIM and complex AIM can further be used. The anti-AIM monoclonal antibody having a property of reacting with free AIM and not reacting with complex AIM and the anti-AIM monoclonal antibody having a property of reacting with both free AIM and complex AIM preferably recognize respective different epitopes.

The anti-AIM monoclonal antibody having a property of reacting with free AIM and not reacting with complex AIM used in the immunoassay method of the present invention binds to an epitope in the SRCR2 domain of human AIM, wherein the epitope has a three-dimensional structure, wherein the SRCR2 domain of human AIM is represented by SEQ ID NO: 3. The anti-AIM monoclonal antibody having a property of reacting with free AIM and not reacting with complex AIM used in the immunoassay method of the present invention preferably does not bind to the SRCR1 domain, and more preferably binds to neither the SRCR1 domain nor the SRCR3 domain.

Although "reacting" with free AIM, "recognizing" free AIM, and "binding" to free AIM are synonymously used in this description, these must be construed in the broadest sense without being limited to these exemplifications. Whether an antibody "reacts" with an antigen (compound) such as free AIM can be confirmed by an antigen solid phase ELISA method, a competitive ELISA method, a sandwich ELISA method, etc. as well as by a method (SPR method) using the principle of surface plasmon resonance etc. The SPR method can be performed by using devices, sensors, and reagents commercially available under the name of Biacore (registered trademark).

Stating that the antibody used "does not react with" a certain compound means that the antibody used in the present invention does not substantially react with a certain compound. Stating "not substantially reacting" means that enhanced reactivity of the antibody used in the present invention is not recognized when Biacore (registered trademark) T100 or T200 is used for immobilizing the antibody of the present invention to perform measurement based on the SPR method, for example. Specifically, this means that the reactivity between the antibody and the compound is not significantly different from the reactivity of a control (with no compound added). Obviously, it can be confirmed that the antibody "does not substantially react" with the compound by a method or means well known to those skilled in the art other than the SPR method.

The binding affinity of the anti-AIM monoclonal antibody specifically reacting with free AIM used in the immunoassay method of the present invention to free AIM is not particularly limited as long as the effect of the present invention can be obtained, and, for example, the binding affinity to IgM can be Kd of at least about 10⁻⁴ M, at least about 10⁻⁵ M, at least about 10⁻⁶ M, at least about 10⁻⁷ M, at least about 10⁻⁸ M, at least about 10⁻⁹ M, at least about 10⁻¹⁰ M, at least about 10⁻¹¹ M, at least about 10⁻¹² M or more.

The antibody used in the immunoassay method of the present invention can be produced by dissolving free AIM as an antigen (immunogen) in a solvent such as phosphate buffered saline and administering this solution to an animal for immunization. The immunization may be performed by using an emulsion after adding an appropriate adjuvant to the solution as needed. The adjuvant may be a widely used adjuvant, such as water-in-oil emulsion, water-in-oil-in-water emulsion, oil-in-water emulsion, liposome, or aluminum hydroxide gel as well as a protein or peptidic substance derived from biogenic components. For example, Freund's incomplete or complete adjuvant can preferably be used. Although not particularly limited, it is desired that the administration route, administered dose, and administration time of the adjuvant are appropriately selected such that a desired immune response can be enhanced in an animal to be immunized by the antigen.

Although not particularly limited, the type of the animal used for the immunization is preferably a mammal and can be a mouse, rat, bovine, rabbit, goat, and sheep, and a mouse or rat is more preferable. The animal may be immunized in accordance with a common technique and, for example, the immunization can be achieved by subcutaneously, intracutaneously, intravenously, or intraperitoneally injecting the animal with a solution of an antigen, preferably a mixture with the adjuvant. Since an immune response is generally different depending on a type and strain of the animal to be immunized, it is desirable that an immunization schedule is appropriately set depending on the animal to be used. The antigen administration is preferably repeated several times after initial immunization.

Although the following operations are continuously performed so as to obtain a monoclonal antibody, the operation is not limited thereto, and a method of producing the monoclonal antibody itself is well known in the art and is widely used, so that those skilled in the art can easily produce the monoclonal antibody used in the immunoassay method of the present invention by using the antigen described above (see, e.g., Antibodies, A Laboratory Manual (Cold Spring Harbor Laboratory Press, (1988), Chapter 6)).

After final immunization, a hybridoma can be produced by extracting spleen or lymph node cells, which are antibody-producing cells, from an immunized animal and by fusing the cells with a myeloma-derived cell line having high proliferative ability. Cells having high antibody-producing ability (quantitative and qualitative) are preferably used for the cell fusion, and the myeloma-derived cell line is preferably compatible with the animal from which the antibody-producing cells to be fused are derived. The cell fusion can be performed in accordance with a method known in the art. For example, a polyethylene glycol method, a method using Sendai virus, or a method utilizing electric current can be employed. The obtained hybridoma can be proliferated in accordance with conditions widely used in the industry. The desired hybridoma can be selected while confirming a property of a produced antibody. The hybridoma can be cloned by a well-known method such as a limiting dilution or soft agar method, for example.

The hybridoma can efficiently be selected at the selection stage in consideration of the conditions under which the produced antibody is actually used in the measurement. For example, an antibody obtained by immunizing an animal is reacted with free AIM immobilized on a solid phase in the presence of a compound (complex AIM) for which cross-reactivity is desired to be confirmed, and the reactivity can be compared with that in the absence of the compound (complex AIM) for which cross-reactivity is desired to be confirmed so as to more efficiently select the hybridoma producing a desired antibody. Alternatively, an antibody obtained by immunizing an animal is reacted with free AIM immobilized on a solid phase in the presence of a biological sample-derived component, and the reactivity can be compared with that in the absence of the biological sample-derived component so as to more efficiently select the hybridoma producing a desired antibody.

After a cloning step, the binding ability of the produced antibody to free AIM can be assayed by using a method such as an ECL method, an ELISA method, a RIA method, and a fluorescent antibody method so as to confirm whether the selected hybridoma produces a monoclonal antibody having a desired property.

A monoclonal antibody having a desired property can be produced by the mass cultivation of the hybridoma selected as described above. Although a method of mass cultivation is not particularly limited, examples thereof can include a method of producing the monoclonal antibody in culture media by cultivating the hybridoma in appropriate culture media. The examples can also include a method of producing the antibody in abdominal dropsy by injecting the hybridoma into the abdominal cavity of a mammal for proliferation. The monoclonal antibody can be purified by appropriately combining the methods for purifying an antibody from the antiserum described above, for example, DEAE anion exchange chromatography, affinity chromatography, an ammonium sulphate fractionation method, a PEG fractionation method, and an ethanol fractionation method.

The monoclonal antibody used in the immunoassay method of the present invention can be a whole antibody molecule as well as a fragment of an antibody having an antigen-antibody reaction activity. The antibody can be obtained through an immunization step of an animal as described above or obtained by a gene recombination technique or can be a chimeric antibody. The fragment of the antibody is preferably a functional fragment, and examples thereof include F(ab')2 and Fab'. These fragments can be produced by processing the antibody obtained as described above with a proteolytic enzyme (e.g., pepsin or papain).

The antibody used in the immunoassay method of the present invention can be used as an immobilized (solid-phased) antibody immobilized on an insoluble carrier or as a labeled antibody labeled with a labeling substance well known to those skilled in the art described later. For example, the immobilized antibody can be produced by causing an insoluble carrier to physically adsorb or chemically bind to a monoclonal antibody (a suitable spacer may exist therebetween). The insoluble carrier can be made of a polymer base material such as a polystyrene resin, an inorganic base material such as glass, and a polysaccharide base material such as cellulose and agarose, and the shape thereof is not particularly limited and can be selected from any shapes such as a plate shape (e.g., microplate and membrane), a bead or particle shape (e.g., latex particles), and a tubular shape (e.g., test tube).

By using a labeled antibody (secondary antibody) that can bind to the antibody used in the immunoassay method of the present invention, an amount of the antibody bound to free AIM can be measured, and free AIM in a biological sample can thereby be detected. Examples of the labeling substance for producing the labeled antibody include enzymes, fluorescent substances, chemiluminescent substances, biotin, avidin, radioisotopes, colloidal gold particles, or colored latex. A method for binding the labeling substance and the antibody can be a method such as a glutaraldehyde method, a maleimide method, a pyridyl disulfide method, or a periodic acid method, which can be used by those skilled in the art, and the types of the immobilized and labeled antibodies and the methods for producing the antibodies are not limited to these examples. For example, when an enzyme such as horseradish peroxidase (HRP) or alkali phosphatase (ALP) is used as the labeling substance, the enzyme activity can be measured by using a specific substrate of the enzyme (e.g., O-phenylenediamine (OPD) or 3,3',5,5'-tetramethylbenzidine (TMB) when the enzyme is HRP, p-nitrophenyl phosphate in the case of ALP). When biotin is used as the labeling substance, at least avidin or enzyme-modified avidin is typically reacted therewith.

In this description, an "insoluble carrier" may be represented as a "solid phase", and physically or chemically supporting an antigen or antibody with an insoluble carrier or the supporting state may be represented as "immobilizing", "immobilized", or "solid phased". The term "assay", "detection", or "measurement" must be construed in the broadest sense, including the existence proof and/or the quantitation of free AIM and must not be construed in a limited manner in any sense.

The immunoassay method of the present invention uses an antibody specifically reacting with at least one free AIM. When performing a so-called sandwich assay in which free AIM to be measured is sandwiched between two antibodies recognizing different epitopes, one of the antibodies may be an antibody specifically reacting with free AIM, and the other antibody may be an anti-AIM monoclonal antibody; however, it is preferable that both of the two antibodies are antibodies specifically reacting with free AIM. When performing a so-called sandwich assay in which free AIM to be measured is sandwiched between two antibodies recognizing different epitopes, the two antibodies preferably recognize respective different epitopes. When the ECL method or the ELISA method is performed, an antibody specifically reacting with free AIM is preferably used as a solid phase antibody immobilized on the solid phase.

### (Immunoassay method)

Examples of the immunoassay method of the present invention include, but not limited to, electrochemiluminescence immunoassay (ECL method), ELISA, enzyme immunoassay, an immunohistochemical staining method, a surface plasmon resonance method, latex agglutination immunoassay, chemiluminescence immunoassay, a fluorescent antibody method, radioimmunoassay, an immunoprecipitation method, a Western Blot method, immunochromatography, the EATA method (Electrokinetic Analyte Transport Assay), and high performance liquid chromatography (HPLC).

By using a labeled antibody (secondary antibody) that can bind to the antibody used, an amount of the antibody bound to free AIM can be measured, and an amount of free AIM in a biological sample can thereby be measured. Examples of a labeling substance for producing the labeled antibody include enzymes, fluorescent substances, chemical luminescent substances, biotin, avidin, radioisotopes, colloidal gold particles, or colored latex. Those skilled in the art can appropriately select an immunoassay method depending on an antibody and a labeling substance used.

The electrochemiluminescence immunoassay (ECL method) is preferably used as the immunoassay method. The electrochemiluminescence immunoassay (ECL method) means a method of calculating an amount of an analyte by causing a labeling substance to emit light by an electrochemical stimulus and detecting an amount of luminescence. In the electrochemiluminescence immunoassay (ECL method), a ruthenium complex can be used as a labeling substance. The amount of luminescence of this ruthenium complex can be detected by disposing an electrode on a solid phase (microplate or beads etc.) and causing an electrochemical stimulus on the electrode.

Although the anti-AIM monoclonal antibody specifically reacting with free AIM may be used as either a solid phase antibody or a detection antibody, the electrochemiluminescence immunoassay (ECL method) is preferably performed by using the anti-AIM monoclonal antibody specifically reacting with free AIM as a solid phase antibody and the anti-AIM monoclonal antibody recognizing an epitope different from the solid phase antibody as a detection antibody (labeled antibody). When an anti-AIM monoclonal antibody specifically reacting with free AIM is used as a solid phase antibody while an anti-AIM monoclonal antibody is used as a labeled antibody, and beads and a ruthenium complex are used as a solid phase and a label, respectively, the measurement principle is as follows. The following describes the measurement principle in an embodiment of the present invention and does not limit the scope of the present invention at all.
1. When the beads having the antibody specifically reacting with free AIM bound thereto are reacted with a sample, the free AIM in the sample binds to the solid phase antibody bound to the beads.
2. After washing the beads, a ruthenium-labeled antibody is reacted with the free AIM bound to the beads and is bound in a sandwich shape.
3. After washing the beads, when electrical energy is applied on the electrode, the ruthenium complex emits light depending on an amount of the ruthenium-labeled antibody bound to the beads via the free AIM. By measuring this amount of luminescence, the free AIM amount in the sample can be measured.

An anti-AIM monoclonal antibody specifically reacting with free AIM and recognizing an epitope different from the solid phase antibody can also be used as the ruthenium-labeled antibody.

Among the immunoassays, the ELISA method using an enzyme label is also preferable since a target can easily and quickly be measured. In the case of sandwich ELISA, an insoluble carrier having an anti-AIM monoclonal antibody specifically reacting with free AIM immobilized thereto and an anti-AIM monoclonal antibody labeled with a labeling substance and recognizing an epitope different from the immobilized antibody can be used. In this case, the insoluble carrier is preferably a plate (immunoplate), and the labeling substance can appropriately be selected and used.

Although the anti-AIM monoclonal antibody specifically reacting with free AIM may be used as either a solid phase antibody or a detection antibody, the sandwich ELISA is preferably performed by using the anti-AIM monoclonal antibody specifically reacting with free AIM as a solid phase antibody and the anti-AIM monoclonal antibody recognizing an epitope different from the solid phase antibody as a detection antibody (labeled antibody). The anti-AIM monoclonal antibody specifically reacting with free AIM immobilized on the insoluble carrier captures the free AIM in the sample and forms an antibody-free AIM complex on the insoluble carrier. The anti-AIM monoclonal antibody labeled with the labeling substance binds to the captured free AIM to form a sandwich with the antibody-free AIM complex described above. The free AIM in the sample can be measured by measuring an amount of the labeling substance by a method corresponding to the labeling substance. For specific methods, such as a method for immobilizing the antibody on the insoluble carrier and a method for binding the antibody and the labeling substance, the methods well known to those skilled in the art can be used without limitation.

An anti-AIM monoclonal antibody specifically reacting with free AIM and recognizing an epitope different from the solid phase antibody can also be used as the labeled antibody.

A latex immunoagglutination method (hereinafter also referred to as an LTIA method) is a typical particle agglutination immunoassay and is also preferable as the immunoassay method. In the LTIA method, latex particles carrying an antibody to a target component are used, and a degree of agglutination (turbidity) of the latex particles caused by binding between an antigen that is the target component and the antibody-supporting latex particles forming an antigen-antibody complex is detected by optical means (e.g., a turbidimetric method for measuring transmitted light, a turbidity method for measuring scattered light), so that the target component can be analyzed. In the immunoassay method of the present invention, latex particles carrying the anti-AIM monoclonal antibody specifically reacting with free AIM are used, and a degree of agglutination of the latex particles caused by binding between free AIM that is the target component and the antibody-supporting latex particles forming an antigen-antibody complex can be detected by optical means. When the LTIA method is adopted, two or more anti-AIM monoclonal antibodies specifically binding to free AIM can be used, or the anti-AIM monoclonal antibody specifically reacting with free AIM and the anti-AIM monoclonal antibody having a property of reacting with both free AIM and complex AIM can also be used.

The high performance liquid chromatography method (HPLC method) or the EATA method (Electrokinetic Analyte Transport Assay) can also be used as the immunoassay method. The EATA method can be performed by using µTAS Wako i30 manufactured by FUJIFILM Wako Pure Chemical Corporation.

### (Assay Kit for Amount of Free AIM)

An assay kit for an amount of free AIM of the present invention includes the anti-AIM monoclonal antibody specifically reacting with free AIM. The assay kit of the present invention can also include other test reagents, specimen diluents, and/or instructions for use.

The assay kit for an amount of free AIM of the present invention can preferably be a free AIM assay kit using the ECL method including (1) and (2) below:
(1) a solid phase on which an anti-AIM monoclonal antibody specifically reacting with free AIM is immobilized; and
(2) an anti-AIM monoclonal antibody labeled with an electrochemical luminescent substance and recognizing an epitope different from the immobilized antibody.

When the ECL method is used, the assay kit of the present invention preferably includes a solid phase on which an antibody specifically reacting with free AIM is immobilized, and an anti-AIM monoclonal antibody labeled with an electrochemical luminescent material such as a ruthenium complex. For example, in the kit using microbeads as the solid phase, a biological sample is added to and reacted with the microbeads on which the antibody specifically reacting with free AIM is solid-phased, and the sample is then removed and washed. Subsequently, an anti-AIM monoclonal antibody labeled with an electrochemical luminescent material and recognizing an epitope different from the antibody specifically reacting with free AIM is added and reacted. After washing the microbeads, electric energy is applied for luminescence, and an amount of luminescence of the labeling substance can be measured to obtain a free AIM concentration.

An anti-AIM monoclonal antibody specifically reacting with free AIM and recognizing an epitope different from the solid phase antibody can also be used as the anti-AIM monoclonal antibody to be labeled with an electrochemical luminescent material.

When the sandwich ELISA method is used, the assay kit can be a free AIM assay kit using the sandwich ELISA method including at least (1) and (2) below:
(1) an insoluble carrier on which an anti-AIM monoclonal antibody (solid phase antibody) specifically reacting with free AIM is immobilized; and
(2) an anti-AIM monoclonal antibody (labeled antibody) labeled with a labeling substance and recognizing an epitope different from the solid phase antibody.

In such a kit, first, a biological sample is added to the insoluble carrier on which the solid phase antibody is immobilized, and is then incubated, and the sample is removed and washed. The labeled antibody is added and then incubated, and a substrate is added for coloring. The free AIM concentration in the biological sample can be obtained by measuring the coloring with a plate reader etc.

An anti-AIM monoclonal antibody specifically reacting with free AIM and recognizing an epitope different from the solid phase antibody can also be used as the labeled antibody.

When the LTIA method is used, the assay kit can be a free AIM assay kit using the LTIA method including at least (1) and (2) below:
(1) latex particles on which an antibody (first solid phase antibody) specifically reacting with free AIM is immobilized; and
(2) latex particles on which an anti-AIM monoclonal antibody (second solid phase antibody) recognizing an epitope different from the solid phase antibody is immobilized.

In such a kit, the first solid phase antibody latex and the second solid phase antibody latex agglutinate via free AIM. The free AIM concentration in the biological sample can be obtained by detecting a degree of agglutination by using optical means.

An anti-AIM monoclonal antibody specifically reacting with free AIM and recognizing an epitope different from the first solid phase antibody can also be used as the second solid phase antibody.

The present invention will hereinafter specifically be described with examples; however, these examples do not limit the scope of the present invention. Unless otherwise described, % denotes % by weight.

### Example

### [Example 1: Production of Antibodies]

### 1. Production of Mouse Anti-Human AIM Monoclonal Antibody

Antibodies Nos. 8, 11, 12, and 29 were mouse anti-human AIM monoclonal antibodies and were obtained by the following procedure.

Emulsion was produced by mixing full-length human rAIM (2 mg/ml) as an antigen with an equal amount of TiterMax Gold (G-1 Funakoshi). Two 8-week-old female Balb/c mice (Charles River Laboratories) were used as immunized animals, and 50 µL of an antigen solution was administered to the sole of the hind foot. The same administration was performed 2 weeks later, and after another 2 weeks or more, 50 µg of the antigen solution was administered to the sole of the hind foot to prepare for cell fusion performed 3 days later.

Mouse P3U1 was used for myeloma cells, and a medium used for growth culture was obtained by adding glutamine and pyruvic acid to RPMI1640 (11875-119 GIBCO) and adding FBS (S1560 BWT) at 10 %. Penicillin and streptomycin were added as antibiotics in appropriate amounts.

Popliteal lymph nodes were aseptically removed from the mice after cardiac blood was collected under anesthesia and were placed on a beaker with #200 mesh and pressed with a silicon rod to prepare a cell suspension. The cells were centrifugally washed twice in RPMI 1640 and then the number of cells was counted. Myeloma cells in the logarithmic growth phase were collected by centrifugation, washed, and then adjusted so that the ratio of lymphocytes to myeloma cells was 5:1, and mixing centrifugation was performed. Cell fusion was performed by using PEG1500 (783641 Roche). Specifically, after a cell pellet was reacted with 1 mL of PEG solution for 3 minutes, then diluted in stages, and washed by centrifugation, a medium was added, and 200 µL was placed in each of 15 96-well plates for 1 week of culture. For the medium, a HAT supplement (21060-017 GIBCO) was added to a medium for myeloma cells to adjust the FBS concentration to 15 %.

After the cryopreserved cells were thawed and proliferation culture was performed, 1×10⁷ cells were administered to the abdominal cavity of a nude mouse (BALB/cAJcl-nu/nu Nippon Claire) to which 0.5 ml of pristane (42-002 Cosmo Bio) was intraperitoneally administered 1 week or more before, and after about 2 weeks, 4 to 12 ml of ascites was obtained. After removing a solid matter by centrifugation, the ascites was cryopreserved. Subsequently, antibodies were purified from the cryopreserved ascites to obtain the antibodies Nos. 8, 11, 12, and 29.

### [Example 2: Screening of Antibody specifically Binding to Free AIM]

### (1. Production of Antibody-Bound Magnetic Beads)

1) The absorbance of the antibody No. 8 dialyzed with 150 mM potassium phosphate buffer (pH 7.8) was measured and adjusted to Abs 0.5 by using the same buffer solution to produce an antibody solution.
2) With the buffer solution, 1 mL of Dynabeads M-450 Epoxy (30 mg/mL) manufactured by Dynamic Biotech was washed 3 times, and 1 mL of the antibody solution produced at 1) was added. Rotary stirring was performed at 25 °C for 18 hours or more.
3) The beads prepared at 2) were washed twice with a bead blocking buffer [50 mM Tris, 150 mM NaCl, 0.1 % BSA (fatty acid free), 0.09 % NaN₃, pH 7.8]. The antibody remaining in the solution and not bound to the beads was removed by removing the buffer solution by washing. Subsequently, 1 mL of the bead blocking buffer was added and stirred, and rotary stirring was performed at 25 °C for 18 hours or more.
4) After washing the beads twice with the bead blocking buffer, 1 mL of the bead blocking buffer was added and stirred. These were used as antibody No. 8-bound magnetic beads.
5) The procedures 1) to 4) were repeated for the antibodies Nos. 11, 12, and 29 to obtain antibody No. 11-bound magnetic beads, antibody No. 12-bound magnetic beads, and antibody No. 29-bound magnetic beads. These four types of antibody-bound magnetic beads were stored at 4 °C until use.

### (2. Production of Ruthenium-Labeled Antibody)

1) To 312.5 µL of an antibody No. 8 solution dialyzed with 150 mM potassium phosphate buffer (pH 7.8), 14.1 µL of 10 mg/mL ruthenium complex (Origin Tag-NHS ESTER manufactured by IGEN) was added, and the solution was stirred for 30 minutes. Subsequently, 50 µL of 2M glycine was added, and the solution was stirred for 20 minutes.
2) A ruthenium complex-labeled antibody produced at 1) was applied to gel filtration column chromatography (Sephadex G-25 manufactured by GE Healthcare Bioscience) packed in a glass tube with a diameter of 1 cm and a height of 30 cm to isolate and purify a non-labeling ruthenium complex and a ruthenium complex-labeled antibody No. 8. Elution was performed with 10 mM potassium phosphate buffer (pH 6.0).
3) The procedures 1) and 2) were repeated for the antibodies Nos. 11, 12, and 29 to obtain a ruthenium complex-labeled antibody No. 11, a ruthenium complex-labeled antibody No. 12, and a ruthenium complex-labeled antibody No. 29.

### (3. Screening of Antibodies Specifically Binding to Free AIM)

1) From NASH-HCC patient serum, 10 µL of a specimen was collected and added to 200 µL of a reaction solution [50 mM HEPES, 50 mM NaCl, 0.05 % Tween 20, 1 mM EDT-4Na, 0.5 % BSA, 0.09 % NaN₃, 100 µg/mL mouse IgG, pH 7.8].
2) To the solution, 25 µL of the antibody No. 11-bound magnetic beads diluted to a concentration of 0.5 mg/mL with a bead diluent [50 mM HEPES, 100 mM NaCl, 0.1 % Tween 20, 1 mM EDT-4Na, 0.5 % BSA, 0.09 % NaN₃, pH 7.8] was added and reacted at 30 °C for 9 minutes (first reaction).
   Subsequently, the magnetic beads were trapped with a magnet, the liquid in the reaction tube was extracted, and the magnetic beads were washed twice with 350 µL of washing liquid [50 mmol/L Tris HCl, 0.01 % (W/V) Tween 20, 0.15 mol/L NaCl, pH 7.5] to remove non-specific binding substances other than the antigen-antibody reaction (BF separation).
3) Subsequently, 200 µL of the ruthenium-labeled antibody No. 12 diluted with a dilute solution for ruthenium [50 mM HEPES, 50 mM NaCl, 0.05 % Tween 20, 1 mM EDT-4Na, 0.5 % BSA, 0.09 % NaN₃, 100 µg/mL mouse IgG, pH 7.8] to a concentration of 0.6 µg/mL was added and reacted at 30 °C for 9 minutes (second reaction).
   The magnetic beads after the reaction were trapped with a magnet, the liquid in the reaction tube was extracted, and the magnetic beads were washed twice with 350 µL of the washing liquid to remove non-specific binding substances other than the antigen-antibody reaction (BF separation).
4) Subsequently, 300 µL of tripropylamine was placed in the reaction tube and mixed with the magnetic beads. By applying electrical energy in this state, the ruthenium complex emitted light, and the emission intensity was detected by a detector.

After the operation of adding the magnetic beads to the reaction tube, this was performed on an automatic ruthenium complex luminescence measuring machine, Picolumi III.

The operation described above was performed in a total of 16 combinations of antibodies (4 types of antibody-bound magnetic beads × 4 types of ruthenium-labeled antibodies) to verify an amount of IgM-AIM included in the measurement in measurement systems.

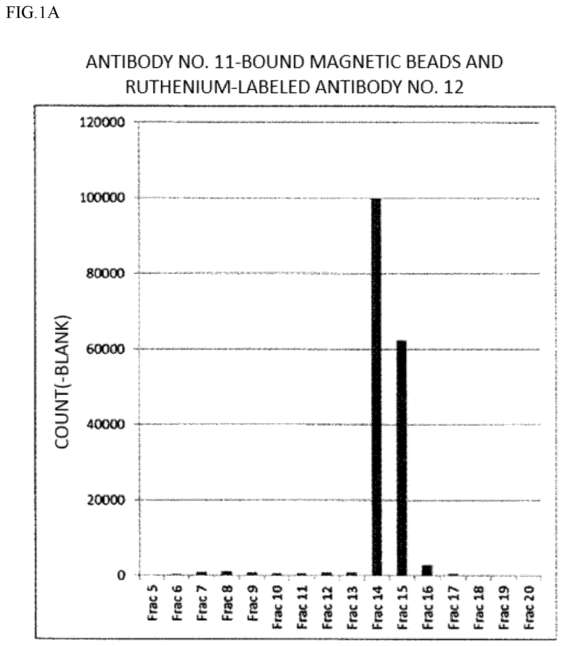

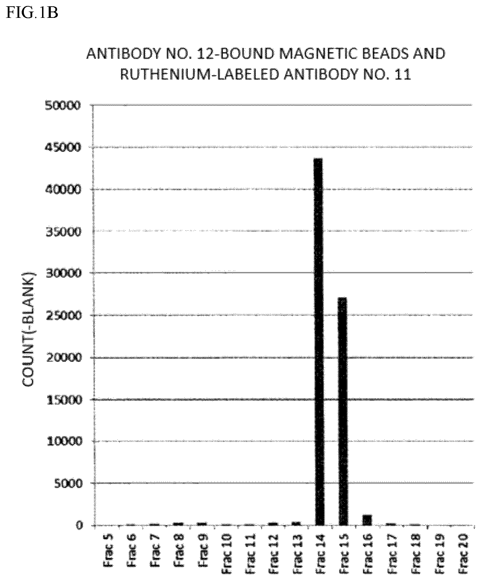

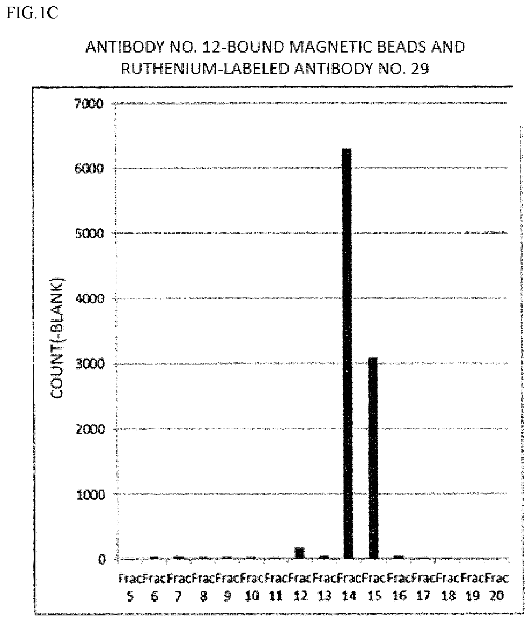

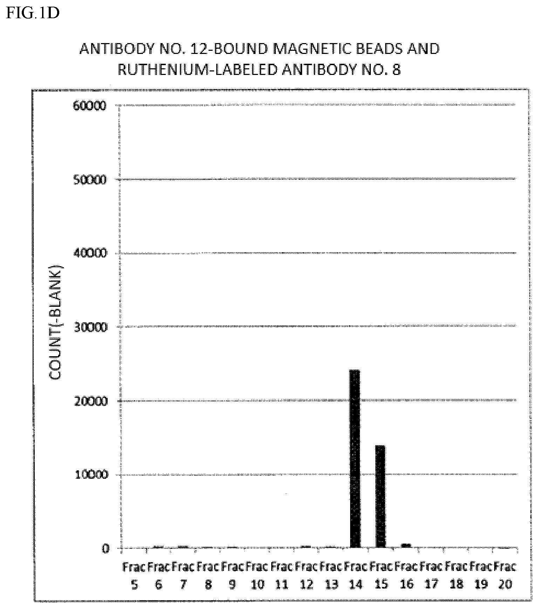

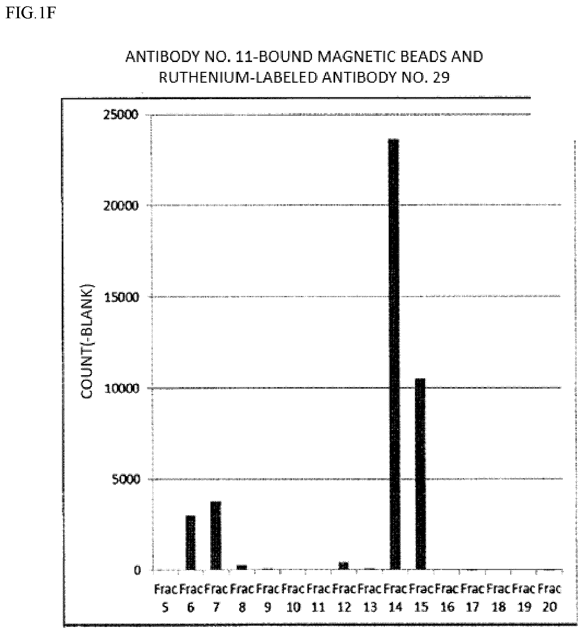

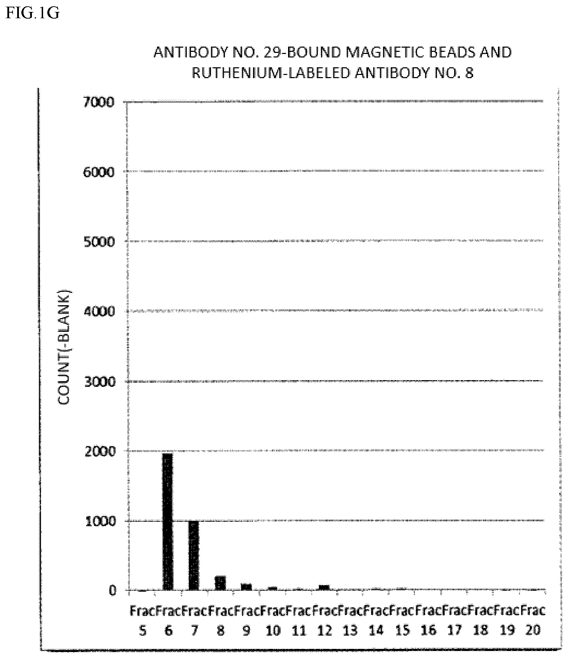

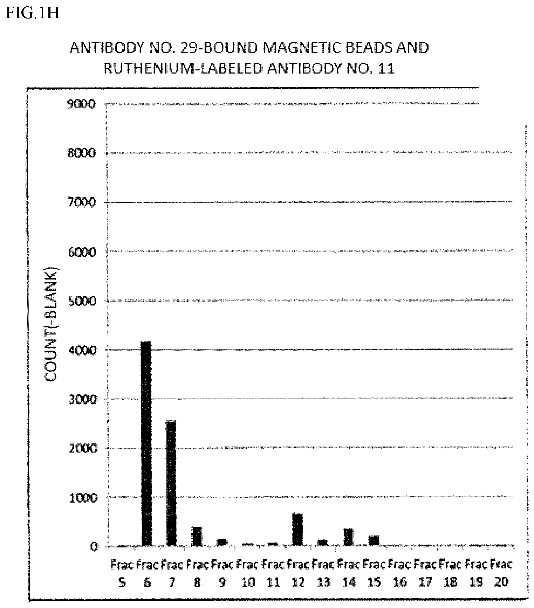

The results are shown in Figs. 1(A) to 1(H). It was found that free AIM can be specifically analyzed when the antibody No. 12 was used for the antibody-bound magnetic beads or the ruthenium-labeled antibody.

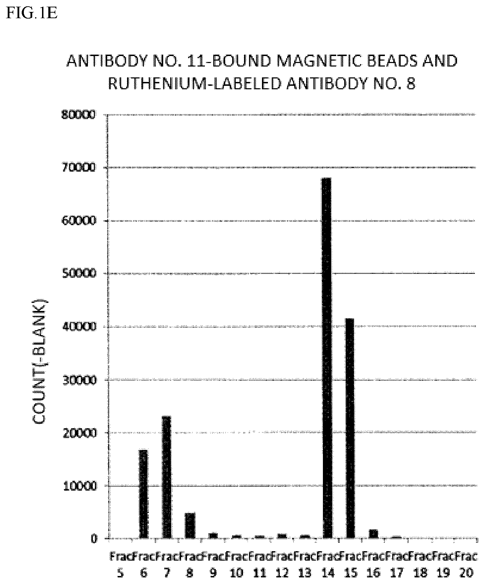

When the antibody No. 12 was not used for the antibody-bound magnetic beads or the ruthenium-labeled antibody, IgM-AIM was included in the measurement in the measurement systems (only 4 measurement systems are shown as Figs. 1(E) to 1(H) and the rest is not shown). Therefore, the antibody No. 12 was demonstrated as a free AIM-specific antibody.

### [Example 3: Epitope Analysis]

### (1. Epitope Analysis by ELISA Method)

The ELISA method was performed by using free AIM (SRCR1+2+3) and SRCR1+2 of AIM (peptide composed of the SRCR1 and SRCR2 domains) as antigens and using the antibody Nos. 8, 11, 12, and 29 as the anti-AIM monoclonal antibodies to analyze the epitopes of the antibody Nos. 8, 11, 12, and 29. Fragments of human AIM used in the experiment were obtained by requesting Takara Bio Inc. to produce them.

The free AIM and the SRCR1+2 of AIM (peptide composed of the SRCR1 domain + SRCR2 domain) were immobilized on a plate, and the antibody Nos. 8, 11, 12, and 29 were added. After washing, an HRP-labeled anti-mouse IgG monoclonal antibody was reacted and colored with an HRP substrate. The results are shown in Tables 1 and 2.

**[Table 1]**

| solid phase | antibody | blank | 1 ng/mL | 10 ng/mL | 100 ng/mL |
|---|---|---|---|---|---|
| SRCR1+2+3 (free AIM) | NO.8 | 0.010 | 0.114 | 0.594 | 1.853 |
| | NO.11 | | 0.126 | 0.790 | 2.164 |
| | NO.12 | | 0.110 | 0.716 | 2.082 |
| | NO.29 | | 0.137 | 0.836 | 2.171 |
| | negative | | 0.011 | 0.012 | 0.025 |
| SRCR 1+2 | NO.8 | 0.007 | 0.009 | 0.010 | 0.023 |
| | NO.11 | | 0.008 | 0.008 | 0.008 |
| | NO.12 | | 0.015 | 0.093 | 0.391 |
| | NO.29 | | 0.008 | 0.009 | 0.011 |
| | negative | | 0.009 | 0.007 | 0.007 |

**[Table 2]**

| | SRCR1+2+3 | SRCR1+2 |
|---|---|---|
| antibody No.8 | + | - |
| antibody No.11 | + | - |
| antibody No.12 | + | + |
| antibody No.29 | + | - |

Table 1 shows ELISA measurement values, and Table 2 shows the reactivity of the antibodies to the antigens based on the ELISA measurement values. "+" denotes being reactive and "-" denotes being non-reactive.

The antibody Nos. 8, 11, and 29 did not react with the peptide composed of the SRCR1 and SRCR2 domains and reacted with free AIM. The antibody No. 12 reacted with both free AIM and the peptide composed of the SRCR1 and SRCR2 domains.

Therefore, it was found from the result of ELISA that the antibody No. 12 recognizes either the SRCR1 domain or the SRCR2 domain as an epitope and that the antibody Nos. 8, 11, and 29 recognize the SRCR3 domain as an epitope.

### (2. Epitope Analysis by Western Blotting)

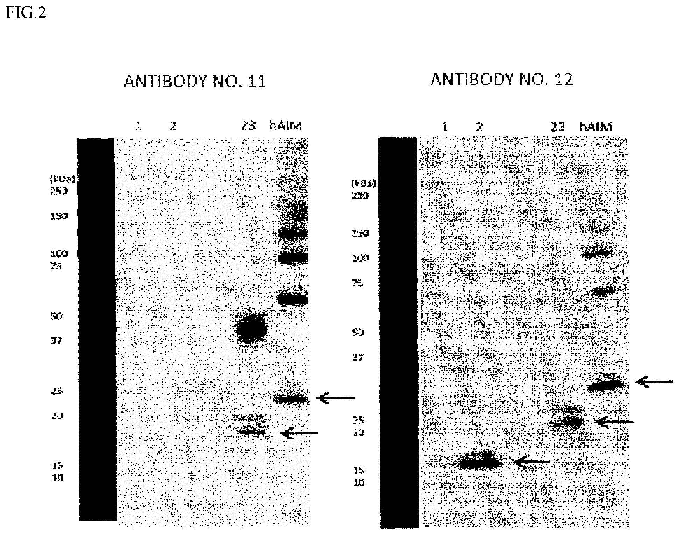

Western blot was performed by using the peptide composed of the SRCR1 domain of AIM ("1" in Fig. 2), the peptide composed of the SRCR2 domain of AIM ("2" in Fig. 2), the peptide composed of the SRCR2 and SRCR3 domains of AIM ("23" in Fig. 2), and human AIM ("hAIM" in Fig. 2) as antigens and using the antibody Nos. 11 and 12 as the anti-AIM monoclonal antibodies to analyze the epitopes of the antibody Nos. 11 and 12. Each of the antigens was mixed with sample buffer and separated by the SDS-PAGE method. After electrophoresis, proteins were transferred to a PVDF membrane (Immobilon, Millipore), and a primary antibody reaction was performed with the antibody Nos. 11 and 12 at 4 °C overnight. An HRP-conjugated anti-mouse IgG antibody was used as a secondary antibody, Luminata Forest Western HRP Substrate (Millipore) was used as a detection reagent, and signals were detected by Image Quant LAS 4000 (GE Healthcare). The results are shown in Fig. 2.

Since the antibody No. 12 reacted with hAIM (SRCR1+2+3), the peptide composed of the SRCR2 and SRCR3 domains, and the peptide composed of the SRCR2, the epitope of the antibody No. 12 was considered to be SRCR2. The antibody No. 12 did not react with SRCR1.

On the other hand, the antibody No. 11 reacted with hAIM (SRCR1+2+3) and the peptide composed of the SRCR2 and SRCR3 and therefore was found to be an antibody reacting with SRCR3. The No. 11 antibody reacted with neither the peptide composed of the SRCR1 nor the peptide composed of the SRCR2.

From the above, it was revealed that the antibody No. 12 recognizes the SRCR2 domain as an epitope and the antibody No. 11 recognizes the SRCR3 domain as an epitope. The SRCRs used in the experiment was obtained by using HEK293 cells, and when HEK293 cells were used, a protein in a state close to the structure in the living body is obtained, and therefore, the antibody No. 12 was considered to be an antibody recognizing the three-dimensional structure in the SRCR2 domain.

Therefore, it was revealed that by screening a monoclonal antibody recognizing the SRCR2 domain as an epitope, the anti-AIM monoclonal antibody reacting with free AIM without reacting with complex AIM can be obtained.

### INDUSTRIAL APPLICABILITY

According to the present invention, only free AIM can specifically be detected in a biological sample containing complex AIM and free AIM.

## Claims

1. An immunoassay method for free AIM in a biological sample comprising:
using an anti-AIM monoclonal antibody having a property of reacting with free AIM and not reacting with complex AIM, wherein the anti-AIM monoclonal antibody is an antibody binding to an epitope in a SRCR2 domain of human AIM, wherein the epitope has a three-dimensional structure, wherein "free AIM" means AIM existing in a free state without being bound to other substances and wherein the SRCR2 domain of human AIM is represented by SEQ ID NO: 3.

2. The immunoassay method according to claim 1, wherein the biological sample is a body fluid sample.

3. The immunoassay method according to claim 2, wherein the body fluid sample is selected from the group consisting of serum, plasma, blood, and urine.

4. The immunoassay method according to any one of claims 1 to 3, wherein an anti-AIM monoclonal antibody having a property of reacting with both free AIM and complex AIM is further used.

5. An anti-AIM monoclonal antibody having a property of reacting with free AIM and not reacting with complex AIM, wherein the monoclonal antibody binds to an epitope in the SRCR2 domain of AIM, wherein the epitope has a three-dimensional structure, wherein "free AIM" means AIM existing in a free state without being bound to other substances and wherein the SRCR2 domain of human AIM is represented by SEQ ID NO: 3.

6. An assay kit for free AIM in a biological sample comprising the monoclonal antibody according to claim 5.

7. The assay kit for free AIM according to claim 6, further comprising an anti-AIM monoclonal antibody having a property of reacting with both free AIM and complex AIM.

## Patentansprüche

1. Immunoassay-Verfahren für freies AIM in einer biologischen Probe, umfassend: Verwenden eines monoklonalen Anti-AIM-Antikörpers, der eine Eigenschaft zum Reagieren mit freiem AIM, nicht jedoch mit komplexem AIM, aufweist, wobei der monoklonale Anti-AIM-Antikörper ein Antikörper ist, der an ein Epitop in einer SRCR2-Domäne von menschlichem AIM bindet, wobei das Epitop eine dreidimensionale Struktur aufweist, wobei "freies AIM" AIM bedeutet, das in einem freien Zustand vorliegt, ohne an andere Substanzen gebunden zu sein, und wobei die SRCR2-Domäne von menschlichem AIM durch die SEQ ID Nr: 3 dargestellt wird.

2. Immunoassay-Verfahren nach Anspruch 1, wobei die biologische Probe eine Körperfluidprobe ist.

3. Immunoassay-Verfahren nach Anspruch 2, wobei die Körperfluidprobe ausgewählt ist aus der Gruppe bestehend aus Serum, Plasma, Blut und Urin.

4. Immunoassay-Verfahren nach einem der Ansprüche 1 bis 3, wobei weiter ein monoklonaler Anti-AIM-Antikörper verwendet wird, der eine Eigenschaft zum Reagieren sowohl mit freiem AIM als auch mit komplexem AIM aufweist.

5. Monoklonaler Anti-AIM-Antikörper, der eine Eigenschaft zum Reagieren mit freiem AIM, nicht jedoch mit komplexem AIM, aufweist, wobei der monoklonale Antikörper an ein Epitop in der SRCR2-Domäne von AIM bindet, wobei das Epitop eine dreidimensionale Struktur aufweist, wobei "freies AIM" AIM bedeutet, das in einem freien Zustand vorliegt, ohne an andere Substanzen gebunden zu sein, und wobei die SRCR2-Domäne von menschlichem AIM durch die SEQ ID Nr: 3 dargestellt wird.

6. Testkit für freies AIM in einer biologischen Probe, umfassend den monoklonalen Antikörper nach Anspruch 5.

7. Testkit für freies AIM nach Anspruch 6, weiter umfassend einen monoklonalen Anti-AIM-Antikörper, der eine Eigenschaft zum Reagieren sowohl mit freiem AIM als auch mit komplexem AIM aufweist.

## Revendications

1. Procédé d'immunoessai d'AIM libre dans un échantillon biologique comprenant : l'utilisation d'un anticorps monoclonal anti-AIM présentant la capacité de réagir avec l'AIM libre et non avec l'AIM complexé, dans lequel l'anticorps monoclonal anti-AIM est un anticorps se liant à un épitope dans un domaine SRCR2 d'AIM humain, dans lequel l'épitope présente une structure tridimensionnelle, dans lequel « AIM libre » signifie que l'AIM existe dans un état libre sans être lié à d'autres substances et dans lequel le domaine SRCR2 d'AIM humain est représenté par la SEQ ID NO : 3.

2. Procédé d'immunoessai selon la revendication 1, dans lequel l'échantillon biologique est un échantillon de fluide corporel.

3. Procédé d'immunoessai selon la revendication 2, dans lequel l'échantillon de fluide corporel est sélectionné parmi le groupe constitué de sérum, de plasma, de sang et d'urine.

4. Procédé d'immunoessai selon l'une quelconque des revendications 1 à 3, dans lequel un anticorps monoclonal anti-AIM présentant la capacité de réagir à la fois avec l'AIM libre et avec l'AIM complexé est en outre utilisé.

5. Anticorps monoclonal anti-AIM présentant la capacité de réagir avec l'AIM libre et non avec l'AIM complexé, dans lequel l'anticorps monoclonal se lie à un épitope dans le domaine SRCR2 d'AIM, dans lequel l'épitope présente une structure tridimensionnelle, dans lequel « AIM libre » signifie que l'AIM existe dans un état libre sans être lié à d'autres substances et dans lequel le domaine SRCR2 d'AIM humain est représenté par la SEQ ID NO : 3.

6. Kit d'analyse pour l'AIM libre dans un échantillon biologique comprenant l'anticorps monoclonal selon la revendication 5.

7. Kit d'analyse pour AIM libre selon la revendication 6, comprenant en outre un anticorps monoclonal anti-AIM présentant la capacité de réagir à la fois avec un AIM libre et un AIM complexé.
